# FASCICULE DE BREVET EUROPEEN

(11) **EP 1 417 952 B1**
(45) Date de publication et mention de la délivrance du brevet: **09.07.2008**
(21) Numéro de dépôt: 03292665.1
(22) Date de dépôt: 24.10.2003
(51) Int. Cl.: A61Q 5/06, A61K 8/04, A61K 8/34, A61K 8/81

(54) **Composition cosmétique capillaire en aérosol à effet de cire**
Haarpflegezusammensetzung mit Wachseffekt in Form eines Aerosols
Hair care aerosol composition providing a wax-like effect

(30) Priorité: 08.11.2002 FR 0214075
(43) Date de publication de la demande: 12.05.2004
(73) Titulaire: L'ORÉAL, 75008 Paris (FR)
(72) Inventeur: de Carvalho, Raquel, 92110 Clichy (FR); Pataut, Françoise, 75017 Paris (FR)
(74) Mandataire: Bourdeau, Françoise

(56) Documents cités:
- WO-A-00/67709
- US-A- 5 320 836

## Description

L'invention concerne un dispositif aérosol contenant une composition cosmétique capillaire, , comprenant des polyols à une concentration élevée ainsi que des polymères fixants anioniques. Elle vise également un procédé cosmétique capillaire comprenant l'application sur les cheveux de cette composition au moyen du dispositif aérosol ainsi que son utilisation pour procurer aux cheveux un effet de cire.

Au sens de la présente invention, on entend par *« composition cosmétique capillaire »*, des compositions de fixation et/ou de maintien des cheveux, des compositions de soin de cheveux, des compositions de conditionnement des cheveux, telles que des compositions destinées à apporter de la douceur aux cheveux ou encore des compositions de maquillage des cheveux.

On entend par *"composition à effet de cire"* une composition pâteuse, destinée à maintenir et/ou à fixer les cheveux entre eux par un certain effet gras, sans le durcissement apporté par les sprays de fixation, tout en respectant la brillance naturelle des cheveux.

La composition cosmétique capillaire distribuée par un dispositif aérosol selon l'invention peut être utilisée dans une application rincée ou non rincée, et de préférence dans une application non rincée.

Les compositions de coiffage, telles que les laques et les sprays, conditionnées sous forme de spray aérosol sont généralement composées d'une phase liquide comprenant, dans un milieu cosmétiquement acceptable alcoolique ou hydroalcoolique, au moins un polymère filmogène, et d'un agent propulseur qui est un gaz liquéfié sous pression réduite ou dissous dans la phase liquide.

Les produits de coiffage à effet de cire se présentent majoritairement sous forme de pâte plus ou moins visqueuse qui s'applique sur la chevelure avec les mains. Généralement, ces produits sont conditionnés dans des tubes ou dans des récipients munis d'un couvercle.

Il serait souhaitable de présenter ce type de produit de coiffage dans un packaging plus simple d'utilisation.

Dans le domaine des produits capillaires, on cherche à fabriquer des laques en aérosol comportant des taux réduits en composés organiques volatils, tel que l'éthanol, et ce pour des raisons essentiellement écologiques, tout en conservant de bonnes propriétés de mise en forme et de maintien de la coiffure.

Le problème posé par l'invention est de trouver des compositions cosmétiques capillaires qui procurent à la chevelure un effet de cire, sans présenter les inconvénients exprimés ci-dessus.

De manière surprenante et inattendue, la Demanderesse a découvert qu'il était possible d'apporter aux cheveux un effet de cire, sans altérer leurs propriétés cosmétiques telle que leur douceur naturelle, en utilisant des polyols en concentration élevée associés à des polymères fixants anioniques, conditionnés sous la forme d'aérosol.

La présente invention a pour objet un dispositif aérosol contenant une composition cosmétique capillaire comprenant :
a) 0,5% à 10 % en poids, par rapport au poids total de la composition aérosol d'au moins un polymère fixant anionique, le polymère fixant anionique étant choisi parmi les homopolymères ou copolymères d'acide acrylique et méthacrylique ou leurs sels, les copolymères d'acide crotonique, les copolymères d'acides ou d'anhydrides carboxyliques monoinsaturés en C₄-C₈, les polyacrylamides à groupements carboxylates, les polyuréthanes anioniques et les polymères siliconés greffés anioniques.
b) au moins un polyol de poids moléculaire inférieur à 500, en concentration supérieure à 15% en poids, par rapport au poids total de la composition aérosol,
c) un milieu hydroalcoolique ou aqueux contenant au moins 10% d'eau en poids, par rapport au poids total de la composition aérosol,
d) au moins un gaz propulseur en concentration supérieure ou égale à 30% en poids, par rapport au poids total de la composition aérosol.

Un autre objet de la présente invention consiste en un procédé de coiffage mettant en oeuvre la composition conditionnée dans un dispositif aérosol.

D'autres objets, caractéristiques, aspects et avantages de l'invention apparaîtront encore plus clairement à la lecture de la description et des divers exemples qui suivent.

Cette association particulière de polymères fixants anioniques et de polyols permet d'obtenir des aérosols à faible teneur en V.O.C., notamment à teneur inférieure ou égale à 55% V.O.C.

Sans être liée par la théorie, la Demanderesse pense que l'effet cireux est obtenu en associant un ou plusieurs polyols de poids moléculaire inférieure à 500, en concentration élevée, et un ou plusieurs polymères fixants anioniques. Les polyols apportent l'aspect « gras » des cires et les polymères fixants anioniques associés apportent la « rigidification du gras » qui permet la mise en forme et le maintien de la coiffure dans la configuration souhaitée ; ceci en l'absence complète de cires dans la formulation.

### POLYMERE FIXANT ANIONIQUE

Les polymères fixants anioniques utilisés sont choisis parmi les polymères suivants :
A) Les homo- ou copolymères d'acide acrylique ou méthacrylique ou leurs sels et en particulier les produits vendus sous les dénominations VERSICOL® E ou K par la société ALLIED COLLOID, et ULTRAHOLD® par la société BASF, les copolymères d'acide acrylique et d'acrylamide vendus sous la forme de leurs sels de sodium sous les dénominations RETEN 421, 423 ou 425 par la Société HERCULES, les sels de sodium des acides polyhydroxycarboxyliques.
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français n° 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevets luxembourgeois n^{os} 75370 et 75371 ou proposés sous la dénomination QUADRAMER par la Société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀, par exemple, de lauryle, tels que celui commercialisé par la société ISP sous la dénomination ACRYLIDONE® LM et les terpolymères acide méthacrylique/acrylate d'éthyle/acrylate de tertiobutyle tels que le produit commercialisé sous la dénomination LUVIMER® 100 P par la société BASF.
   On peut aussi citer les copolymères acide méthacrylique/acide acrylique/acrylate d'éthyle/méthacrylate de méthyle en dispersion aqueuse, commercialisé sous la dénomination AMERHOLD® DR 25 par la société AMERCHOL.
C) Les copolymères d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés ou réticulés, ou encore un autre monomère ester vinylique, allylique ou méthallylique d'un acide carboxylique alpha- ou alpha-cyclique. De tels polymères sont décrits entre autres dans les brevets français n^{os} 1 222 944, 1 580 545, 2 265 782, 2 265 781, 1 564 110 et 2 439 798. Des produits commerciaux entrant dans cette classe sont les résines 28-29-30, 26-13-14 et 28-13-10 commercialisées par la société National Starch.
D) Les copolymères d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US n^{os} 2 047 398, 2 723 248, 2 102 113, le brevet GB n° 839 805. Des produits commerciaux sont notamment ceux vendus sous les dénominations GANTREZ® AN ou ES par la société ISP.
   - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, alpha-oléfine, esters acryliques ou méthacryliques, acides acrylique ou méthacrylique ou vinylpyrrolidone dans leur chaîne,
   les fonctions anhydrides de ces copolymères étant éventuellement monoestérifiées ou monoamidifiées.
   Ces polymères sont par exemple décrits dans les brevets français n^{os} 2 350 384 et 2 357 241 de la demanderesse.
E) Les polyacrylamides comportant des groupements carboxylates.
   Les homopolymères et copolymères comprenant des groupements sulfoniques sont des polymères comportant des motifs vinylsulfonique, styrène-sulfonique, naphtalène-sulfonique ou acrylamido-alkylsulfonique.
   Ces polymères peuvent être notamment choisis parmi :
   - les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone.
   - les sels de l'acide polystyrène-sulfonique tels que les sels de sodium vendus par exemple sous les dénominations Flexan® 500 et Flexan® 130 par National Starch.
      Ces composés sont décrits dans le brevet FR 2 198 719.
   - les sels d'acides polyacrylamide-sulfoniques tels que ceux mentionnés dans le brevet US 4 128 631, et plus particulièrement l'acide polyacrylamidoéthylpropane-sulfonique vendu sous la dénomination COSMEDIA POLYMER HSP 1180 par Henkel.
      Les polymères anioniques de l'invention peuvent être également des polycondensats comprenant au moins une séquence polyuréthane, ainsi qu'au moins un groupement anionique. Ces polycondensats peuvent ou non comprendre une séquence polysiloxane. Parmi ces polycondensats, on préfère utiliser les polyuréthanes anioniques, et en particulier, ceux commercialisés par BASF sous la dénomination Luviset PUR ou Luviset Si PUR.
      Les polymères anioniques de l'invention peuvent aussi être des polymères à squelette siliconé et à greffons hydrocarbonés et des polymères à squelette hydrocarboné et à greffons siliconé, ces polymères devant comporter dans leur structure, au moins un groupement anionique. De préférence, les greffons sont fixés au squelette via un macromonomère. Comme polymère de ce type, on peut utiliser le polymère VS30de la Société 3M.

Selon l'invention, le polymère fixant anionique est choisi parmi les homopolymères ou copolymères d'acide acrylique et méthacrylique ou leurs sels, les copolymères d'acide crotonique, les copolymères d'acides ou d'anhydrides carboxyliques monoinsaturés en C₄-C₈, les polyacrylamides à groupements carboxylates, les homopolymères ou copolymères à groupes sulfoniques, les polyuréthanes anioniques, et les polymères siliconés greffés anioniques.

La composition de coiffage contenue dans le dispositif selon l'invention présente une teneur en polymères fixants anioniques comprise de préférence entre 0,5 et 10 % en poids, mieux encore entre 1 et 8 % en poids, et encore plus préférentiellement entre 2 et 6 % en poids par rapport au poids total de ladite composition.

### POLYOL

Les polyols utilisés dans les dispositifs de l'invention sont des molécules comportant une chaîne hydrocarbonnée en C3 à C30, interrompue ou non par un ou plusieurs hétéroatomes et au moins substituée par deux groupements hydroxy.

La composition de coiffage contenue dans le dispositif selon l'invention présente une teneur en polyols comprise de préférence entre 15 et 55 % en poids, mieux encore entre 15 et 40 % en poids, et encore plus préférentiellement entre 20 et 35 % en poids par rapport au poids total de ladite composition.

Parmi les polyols utilisables selon l'invention, on peut citer, de préférence, le propylène glycol, le glycérol (glycérine), l'isoprène glycol, le néopentyl glycol, l'hexylène glycol, les polyéthylènes glycol.

De préférence, la chaîne hydrocarbonée n'est pas interrompue par un hétéroatome, encore plus préférentiellement, le nombre d'atomes de carbone de la chaîne hydrocarbonnée est inférieur à 10, et encore plus préférentiellement inférieur à 8.

Le propylène glycol et la glycérine sont particulièrement préférés.

Préférentiellement, le polyol est un glycol, c'est-à-dire qu'il comporte au moins deux atomes de carbone adjacents, porteur chacun d'au moins un groupement hydroxy.

### MILIEU HYDROALCOOLIQUE

La composition de coiffage contenue dans le dispositif selon l'invention présente une teneur en eau comprise de préférence entre 10 et 54,5 % en poids, mieux encore entre 10 et 45 % en poids, et encore plus préférentiellement entre 10 et 30 % en poids par rapport au poids total de ladite composition.

### PROPULSEUR

Conformément à l'invention, on utilise, de préférence, comme gaz propulseur, au moins un gaz soluble ou non dans la composition tel que le diméthyl éther, les hydrocarbures fluorés ou non, les gaz liquéfiés usuels ou un mélange de ces gaz propulseurs. De préférence, on utilise le diméthyl éther.

La composition de coiffage contenue dans le dispositif selon l'invention présente une teneur gaz propulseur comprise de préférence entre 30 et 50 % en poids, mieux encore entre 30 et 45 % en poids, et encore plus préférentiellement entre 30 et 40 % en poids par rapport au poids total de ladite composition.

De préférence, la composition contient, comme alcool, de l'éthanol ou de l'isopropanol.

La composition de coiffage peut comprendre en outre des additifs tels que des silicones sous forme soluble, dispersée, micro-dispersée, des actifs traitants, des hydratants autres que les polyols de l'invention, des filtres UV, des acides, des bases, des agents plastifiants, des agents solubilisants, des agents conservateurs, des vitamines et des provitamines, des colorants, des pigments, des agents tensioactifs anioniques, cationiques, non ioniques ou amphotères, des polymères fixants cationiques, non ioniques ou amphotères, des parfums, des agents anti-corrosion, et leurs mélanges.

L'homme de métier veillera à choisir les éventuels additifs et leur quantité de manière à ce qu'ils ne nuisent pas aux propriétés des compositions de la présente invention.

L'invention pourra être mieux comprise à l'aide de l'exemple non limitatif qui suit et qui constitue un mode de réalisation préférentiel des compositions selon l'invention.

### Exemple:

On réalise un spray aérosol 55% VOC comprenant en g/100g de matière active :
- PVP/VA : 1
- Ultra Hold Strong (BASF) : 4
- Aminomethyl propanol : 0.48
- Propylène Glycol : 15
- Glycérine : 15
- Parfum : 0.2
- Eau: 10.32
- Alcool : 14
- DME : 40

PVP/VA: commercialisé par BASF sous l'appellation Luviskol VA 64 Poudre

## Revendications

1. Dispositif aérosol contenant une composition cosmétique capillaire, comprenant :
a)0,5% à 10 % en poids, par rapport au poids total de la composition aérosol d'au moins un polymère fixant anionique, le polymère fixant anionique étant choisi parmi les homopolymères ou copolymères d'acide acrylique et méthacrylique ou leurs sels, les copolymères d'acide crotonique, les copolymères d'acides ou d'anhydrides carboxyliques monoinsaturés en C₄-C₈, les polyacrylamides à groupements carboxylates, les polyuréthanes anioniques, et les polymères siliconés greffés anioniques,
b)au moins un polyol, de poids moléculaire inférieur à 500, en concentration supérieure à 15% en poids, par rapport au poids total de la composition aérosol,
c)un milieu hydroalcoolique ou aqueux contenant au moins 10% d'eau en poids, par rapport au poids total de la composition aérosol,
d)au moins un gaz propulseur en concentration supérieure ou égale à 30% en poids, par rapport au poids total de la composition aérosol.

2. Dispositif selon la revendication 1, **caractérisé en ce que** la teneur en polymères fixants anioniques est comprise entre 0,5 et 10 % en poids, mieux encore entre 1 et 8 % en poids, et encore plus préférentiellement entre 2 et 6 % en poids par rapport au poids total de ladite composition.

3. Dispositif selon l'une quelconque des revendications précédentes, **caractérisé en ce que** la teneur en glycols est comprise entre 15 et 55 % en poids, mieux encore entre 15 et 40 % en poids, et encore plus préférentiellement entre 20 et 35 % en poids par rapport au poids total de ladite composition.

4. Dispositif selon l'une quelconque des revendications précédentes, **caractérisé en ce que** le polyol est choisi parmi le propylène glycol, le glycérol (glycérine), l'isoprène glycol, le néopentyl glycol, l'hexylène glycol, les polyéthylènes glycol.

5. Disposition selon l'une quelconque des revendications précédentes, **caractérisé en ce que** le polyol contient une chaîne hydrocarbonée qui n'est pas interrompue par un hétéroatome, encore plus préférentiellement, le nombre d'atomes de carbone de la chaîne hydrocarbonnée est inférieur à 10, et encore plus préférentiellement inférieur à 8.

6. Dispositif selon l'une quelconque des revendications précédentes, **caractérisé en ce que** le polyol est un glycol, c'est-à-dire qu'il comporte au moins deux atomes de carbone adjacents, porteur chacun d'au moins un groupement hydroxy.

7. Dispositif selon l'une quelconque des revendications précédentes, **caractérisé en ce que** le polyol est choisi parmi le propylène glycol et la glycérine.

8. Dispositif selon l'une quelconque des revendications précédentes, **caractérisé en ce qu'**il contient, comme alcool, de l'éthanol ou de l'isopropanol.

9. Dispositif selon l'une quelconque des revendications précédentes, **caractérisé en ce que** la teneur en eau est comprise entre 10 et 54,5 % en poids, mieux encore entre 10 et 45 % en poids, et encore plus préférentiellement entre 10 et 30 % en poids par rapport au poids total de ladite composition.

10. Dispositif selon l'une quelconque des revendications précédentes, **caractérisé en ce que** la teneur en gaz propulseur est comprise de préférence entre 30 et 50 % en poids, mieux encore entre 30 et 45 % en poids, et encore plus préférentiellement entre 30 et 40 % en poids par rapport au poids total de ladite composition.

11. Dispositif selon l'une quelconque des revendications précédentes, **caractérisé en ce que** le gaz propulseur est le diméthyl éther

12. Dispositif selon l'une quelconque des revendications précédentes, **caractérisé en ce que** la composition comprend, en outre, des additifs tels que des silicones sous forme soluble, dispersée, micro-dispersée, des actifs traitants, des hydratants autres que les polyols de l'invention, des filtres UV, des acides, des bases, des agents plastifiants, des agents solubilisants, des agents conservateurs, des vitamines et des provitamines, des colorants, des pigments, des agents tensioactifs anioniques, cationiques, non ioniques ou amphotères, des polymères fixants cationiques, non ioniques ou amphotères, des parfums, des agents anti-corrosion, et leurs mélanges.

13. Procédé cosmétique capillaire comprenant l'application sur les cheveux d'une composition distribuée par un dispositif aérosol selon l'une quelconque des revendications précédentes.

14. Utilisation d'un dispositif selon l'une quelconque des revendications 1 à 12, pour procurer aux cheveux un effet de cire.

## Claims

1. Aerosol device containing a cosmetic composition for the hair, comprising:
a) 0.5% to 10% by weight, based on the total weight of the aerosol composition, of at least one anionic fixing polymer, the anionic fixing polymer being selected from homopolymers or copolymers of acrylic and methacrylic acid or their salts, crotonic acid copolymers, copolymers of monounsaturated C₄-C₈ carboxylic acids or anhydrides, polyacrylamides with carboxylate groups, anionic polyurethanes and anionic grafted silicone polymers.
b) at least one polyol with a molecular weight below 500, in a concentration above 15% by weight, based on the total weight of the aerosol composition,
c) an aqueous-alcoholic or aqueous medium containing at least 10% by weight of water, based on the total weight of the aerosol composition, and
d) at least one propellant gas in a concentration greater than or equal to 30% by weight, based on the total weight of the aerosol composition.

2. Device according to Claim 1, **characterized in that** the content of anionic fixing polymers is between 0.5 and 10% by weight, preferably between 1 and 8% by weight and particularly preferably between 2 and 6% by weight, based on the total weight of said composition.

3. Device according to either of the preceding claims, **characterized in that** the glycol content is between 15 and 55% by weight, preferably between 15 and 40% by weight and particularly preferably between 20 and 35% by weight, based on the total weight of said composition.

4. Device according to any one of the preceding claims, **characterized in that** the polyol is selected from propylene glycol, glycerol, isoprene glycol, neopentyl glycol, hexylene glycol and polyethylene glycols.

5. Device according to any one of the preceding claims, **characterized in that** the polyol contains a hydrocarbon chain which is not interrupted by a heteroatom, the number of carbon atoms in the hydrocarbon chain being preferably less than 10 and particularly preferably less than 8.

6. Device according to any one of the preceding claims, **characterized in that** the polyol is a glycol, i.e. it contains at least two adjacent carbon atoms each carrying at least one hydroxyl group.

7. Device according to any one of the preceding claims, **characterized in that** the polyol is selected from propylene glycol and glycerol.

8. Device according to any one of the preceding claims, **characterized in that** it contains ethanol or isopropanol as the alcohol.

9. Device according to any one of the preceding claims, **characterized in that** the water content is between 10 and 54.5% by weight, preferably between 10 and 45% by weight and particularly preferably between 10 and 30% by weight, based on the total weight of said composition.

10. Device according to any one of the preceding claims, **characterized in that** the propellant gas content is preferably between 30 and 50% by weight, particularly preferably between 30 and 45% by weight and very particularly preferably between 30 and 40% by weight, based on the total weight of said composition.

11. Device according to any one of the preceding claims, **characterized in that** the propellant gas is dimethyl ether.

12. Device according to any one of the preceding claims, **characterized in that** the composition also comprises additives such as silicones in soluble, dispersed or microdispersed form, treating agents, hydrating agents other than the polyols of the invention, UV filters, acids, bases, plasticizers, solubilizers, preservatives, vitamins and provitamins, colorants, pigments, anionic, cationic, non-ionic or amphoteric surfactants, cationic, non-ionic or amphoteric fixing polymers, perfumes, corrosion inhibitors and mixtures thereof.

13. Cosmetic method for the hair comprising the application to the hair of a composition dispensed by an aerosol device according to any one of the preceding claims.

14. Use of a device according to any one of Claims 1 to 12 for imparting a waxy effect to the hair.

## Patentansprüche

1. Aerosolvorrichtung, die eine haarkosmetische Zusammensetzung enthält, enthaltend:
a) 0,5 bis 10 Gew.-% mindestens eines anionischen festigenden Polymers, bezogen auf das Gesamtgewicht der Aerosolzusammensetzung, wobei das anionische festigende Polymer unter den Homopolymeren oder Copolymeren von Acrylsäure und Methacrylsäure oder deren Salzen, Copolymeren von Crotonsäure, Copolymeren von einfach ungesättigten C₄₋₈-Carbonsäuren oder C₄₋₈-Carbonsäureanhydriden, Polyacrylamiden mit Carboxylatgruppen, anionischen Polyurethanen und anionischen gepfropften Siliconpolymeren ausgewählt ist,
b) mindestens ein Polyol mit einem Molekulargewicht unter 500 in einer Konzentration über 15 Gew.-%, bezogen auf das Gesamtgewicht der Aerosolzusammensetzung,
c) ein wässrig-alkoholisches oder wässriges Medium, das mindestens 10 Gew.-% Wasser, bezogen auf das Gesamtgewicht der Aerosolzusammensetzung, enthält,
d) mindestens ein Treibmittel in einer Konzentration von 30 Gew.-% oder darüber, bezogen auf das Gesamtgewicht der Aerosolzusammensetzung.

2. Vorrichtung nach Anspruch 1, **dadurch gekennzeichnet, dass** der Mengenanteil der anionischen festigenden Polymere im Bereich von 0,5 bis 10 Gew.-%, besser 1 bis 8 Gew.-% und noch bevorzugter 2 bis 6 Gew.-%, bezogen auf das Gesamtgewicht der Zusammensetzung, liegt.

3. Vorrichtung nach einem der vorhergehenden Ansprüche, **dadurch gekennzeichnet, dass** der Gehalt an Glycolen im Bereich von 15 bis 55 Gew.-%, besser 15 bis 40 Gew.-% und noch bevorzugter 20 bis 35 Gew.-%, bezogen auf das Gesamtgewicht der Zusammensetzung, liegt.

4. Vorrichtung nach einem der vorhergehenden Ansprüche, **dadurch gekennzeichnet, dass** das Polyol unter Propylenglycol, Glycerol (Glycerin), Isoprenglycol, Neopentylglycol, Hexylenglycol, Polyethylenglycolen ausgewählt ist.

5. Vorrichtung nach einem der vorhergehenden Ansprüche, **dadurch gekennzeichnet, dass** das Polyol eine Kohlenwasserstoffkette aufweist, die nicht durch ein Heteroatom unterbrochen ist, wobei noch bevorzugter die Anzahl der Kohlenstoffatome der Kohlenwasserstoffkette unter 10 und noch bevorzugter unter 8 liegt.

6. Vorrichtung nach einem der vorhergehenden Ansprüche, **dadurch gekennzeichnet, dass** das Polyol ein Glycol ist, d. h. dass es mindestens 2 aneinander angrenzende Kohlenstoffatome aufweist, die jedes mindestens eine Hydroxygruppe tragen.

7. Vorrichtung nach einem der vorhergehenden Ansprüche, **dadurch gekennzeichnet, dass** das Polyol unter Propylenglycol und Glycerin ausgewählt ist.

8. Vorrichtung nach einem der vorhergehenden Ansprüche, **dadurch gekennzeichnet, dass** sie als Alkohol Ethanol oder Isopropanol enthält.

9. Vorrichtung nach einem der vorhergehenden Ansprüche, **dadurch gekennzeichnet, dass** der Wassergehalt im Bereich von 10 bis 54,5 Gew.-%, besser 10 bis 45 Gew.-% und noch bevorzugter 10 bis 30 Gew.-%, bezogen auf das Gesamtgewicht der Zusammensetzung, liegt.

10. Vorrichtung nach einem der vorhergehenden Ansprüche, **dadurch gekennzeichnet, dass** der Gehalt an Treibmittel vorzugsweise im Bereich von 30 bis 50 Gew.-%, besser 30 bis 45 Gew.-% und noch bevorzugter 30 bis 40 Gew.-%, bezogen auf das Gesamtgewicht der Zusammensetzung, liegt.

11. Vorrichtung nach einem der vorhergehenden Ansprüche, **dadurch gekennzeichnet, dass** das Treibmittel der Dimethylether ist.

12. Vorrichtung nach einem der vorhergehenden Ansprüche, **dadurch gekennzeichnet, dass** die Zusammensetzung ferner Zusatzstoffe enthält, wie Silicone in löslicher, dispergierter, mikrodispergierter Form, Behandlungsmittel, Hydratisierungsmittel, die von den erfindungsgemäßen Polyolen verschieden sind, UV-Filter, Säuren, Basen, Weichmacher, Lösevermittler, Konservierungsmittel, Vitamine, Provitamine, Farbmittel, Pigmente, anionische, kationische, nichtionische oder amphotere grenzflächenaktive Stoffe, kationische, nichtionische oder amphotere festigende Polymere, Parfums, Korrosionsschutzmittel und deren Gemische.

13. Kosmetisches Verfahren für die Haarbehandlung, das das Aufbringen einer aus einer Aerosolvorrichtung nach einem der vorhergehenden Ansprüche abgegebenen Zusammensetzung auf die Haare umfasst.

14. Verwendung einer Vorrichtung nach einem der Ansprüche 1 bis 12, um den Haaren einen Wachseffekt zu geben.
